# EUROPÄISCHE PATENTSCHRIFT

(11) **EP 1 473 068 B1**
(45) Veröffentlichungstag und Bekanntmachung des Hinweises auf die Patenterteilung: **10.01.2007**
(21) Anmeldenummer: 04008167.1
(22) Anmeldetag: 03.04.2004
(51) Int. Cl.: B01D 3/32, B01J 19/32, C07D 487/08

(54) **Verfahren zur destillativen Gewinnung von hochreinem Triethylendiamin (TEDA)**
Process for the preparation of pure triethylendiamine (TEDA)
Procédé de préparation de triethylenediamine pur (TEDA)

(30) Priorität: 29.04.2003 DE 10319159
(43) Veröffentlichungstag der Anmeldung: 03.11.2004
(73) Patentinhaber: BASF Aktiengesellschaft, 67056 Ludwigshafen (DE)
(72) Erfinder: Ortmund, Lang, 66909 Quirnbach (DE); Rumpf, Bernd, Dr., 68766 Hockenheim (DE); Frauenkron, Matthias, Dr., 67251 Freinsheim (DE); Bosch, Marco, Dr., 68159 Mannheim (DE); Berrsche, Helmut, Dr., 67454 Hassloch (DE); Meier, Anton, Dr., 67134 Birkenheide (DE)

(56) Entgegenhaltungen:
- DE-A- 19 933 850
- US-A- 3 297 701
- SCHULTZ M A ET AL: "Reducing Costs with Dividing-Wall Columns" CEP MAGAZINE, [Online] Bd. 98, Nr. 5, 2002, Seiten 64-71, XP002291891 Gefunden im Internet: URL:http://www.cepmagazine.org/050264.pdf> [gefunden am 2004-08-10]
- LESTAK ET AL: "ADVANCED DISTILLATION SAVESE" CHEMICAL ENGINEERING, MCGRAW-HILL, ALBANY, NY, US, Bd. 7, Juli 1997 (1997-07), Seiten 72-76, XP001156299 ISSN: 0009-2460
- KAIBEL G: "DISTILLATION COLUMNS WITH VERTICAL PARTITIONS" CHEMICAL ENGINEERING AND TECHNOLOGY, WEINHEIM, DE, Bd. 10, 1987, Seiten 92-98, XP002939161 ISSN: 0930-7516

## Beschreibung

Die vorliegende Erfindung betrifft ein verbessertes Verfahren zur destillativen Gewinnung von Triethylendiamin (= TEDA = DABCO® = 1,4-Diazabicyclo-[2,2,2]-octan) und Lösungen hiervon.

TEDA , das unter Normalbedingungen ein Feststoff ist, ist ein bedeutender Katalysator für die Herstellung von Polyurethanschäumen.

Für diese und andere Einsatzgebiete ist ein reines, möglichst geruchsloses und reinweißes TEDA mit einer möglichst geringen Verfärbung, z.B. mit einer möglichst kleinen APHA-Farbzahl (DIN-ISO 6271), das diese Eigenschaften auch über längere Lagerzeiten (von z.B. 6, 12 oder mehr Monaten) beibehält, erwünscht.

Zur Herstellung und Reinigung von TEDA sind verschiedene Verfahren bekannt. In der DE-A 19962455 wird ein Verfahren zur Herstellung von TEDA beschrieben, bei welchem TEDA verdampft wird und das dampfförmige TEDA in ein flüssiges Lösungsmittel eingeleitet wird.

In der DE-A 10100943 wird ein Verfahren zur Herstellung von TEDA offenbart, bei welchem u.a. das Lösungs- bzw. Verdünnungsmittel spezielle Eigenschaften aufweist.

In der DE-A 19933850 wird ein Verfahren zur Reinigung von TEDA beschrieben, bei welchem man das TEDA nach einer fraktionierenden Destillation in ein flüssiges Lösungsmittel einleitet.

Bei der großtechnischen Herstellung wird TEDA aus Roh-TEDA üblicherweise in diskontinuierlich betriebenen Destillationseinrichtungen oder in zwei oder mehreren kontinuierlich betriebenen Destillationseinrichtungen aufgearbeitet. Dies wird beispielsweise in der DE-A 19933850 (Seite 3, Zeilen 30 bis 43) beschrieben.

Die nach der Vorabtrennung der Leicht- und Schwersieder noch enthaltenen Nebenkomponenten werden bei der konventionellen Destillation in der Reindestillation zum Teil jedoch zersetzt und können zur Bildung von unerwünschten Nebenprodukten führen, wodurch die Produktqualität des TEDA verschlechtert werden kann. Somit ist - um den üblichen hohen Anforderungen bei der Produktqualität hinsichtlich Parametern wie Farbe, Farbstabilität, Geruch und Produktreinheit - ein großer verfahrenstechnischer Aufwand nötig, um den geschilderten Nachteilen zu begegnen.

Es stellte sich somit die Aufgabe, ein verbessertes Verfahren zur Reinigung von Roh-TEDA zu finden, welches die Gewinnung von hochreinem TEDA in hoher Qualität in verfahrenstechnisch einfacher und kostengünstiger Art und Weise ermöglicht.

Demgemäss wurde ein Verfahren zur destillativen Reinigung von Triethylendiamin (TEDA) gefunden, welches dadurch gekennzeichnet ist, dass man die Auftrennung in einer Trennwandkolonne durchführt, deren innen liegendes Trennblech nach unten bis in den Sumpf geführt wird, sodass im unteren Kolonnenbereich getrennte Räume ausgebildet werden.

Trennwandkolonnen sind allgemein bekannte Destillationskolonnen und in der Literatur umfangreich beschrieben. Sie weisen senkrechte Trennwände auf, die in Teilbereichen eine Quervermischung von Flüssigkeits- und Brüdenströmen verhindern. Meist besteht die Trennwand aus einem ebenen Blech und sie unterteilt die Kolonne in Längsrichtung in deren mittlerem Bereich in einen Zulaufteil und einen Entnahmeteil. Bei dem erfindungsgemäßen Verfahren wird das aufzutrennende Gemisch, das Roh-TEDA, dem Zulaufteil der Trennwandkolonne zugeführt und das Produkt, das Rein-TEDA wird aus dem Entnahmeteil flüssig oder dampfförmig abgezogen.

Der Einsatz einer Trennwandkolonne wäre bei dem vorliegenden Stoffsystem vom Fachmann nicht in Betracht gezogen worden, da - wie bereits vorstehend erläutert - aufgrund von stofflichen Zersetzungsprozessen unerwünschte Nebenprodukte gebildet werden, welche die Produktqualität verschlechtern können. Daher wurde bei diesem Stoffsystem wegen der allgemein geforderten, hohen Qualität an TEDA nur eine zweistufige Destillation als aussichtsreich eingeschätzt. Im Fall einer Trennwandkolonne wären in dieser Hinsicht verstärkte Nachteile zu erwarten gewesen. Es stellte sich jedoch heraus, dass durch das erfindungsgemäße Verfahren hochreines TEDA in verfahrenstechnisch einfacher und wirtschaftlicher Weise gewonnen werden kann.

Generell ist bei dem erfindungsgemäßen Verfahren die Trennwand in Kolonnenlängsrichtung unter Ausbildung eines oberen gemeinsamen Kolonnenbereichs und/oder eines unteren gemeinsamen Kolonnenbereichs, eines Zulaufteils und eines Entnahmeteils angeordnet und die Trennwand nach unten bis in den Sumpf der Kolonne geführt, sodass nur im oberen Bereich der Kolonne ein gemeinsamer Bereich ausgebildet wird. Hierdurch wird vorteilhafterweise erreicht, dass durch Zersetzung der Schwersieder oder Nebenkomponenten entstehende Leichtsieder nur in dem unteren Kolonnenteil nach oben geführt werden, welcher dem Kolonnenzulauf zugeordnet ist. Hierdurch wird ein unerwünschter Austrag dieser Leichtsieder über den Seitenabzug vermieden.

Das Verfahren wird in der Regel kontinuierlich durchgeführt.

Die Trennwandkolonne ist bevorzugt mit zwei Sumpfverdampfern und einem Kondensator am Kolonnenkopf ausgestattet.

Bei dem erfindungsgemäßen Verfahren wird in vorteilhafter Weise die Verweilzeit im Sumpfverdampfer und dem zugehörigen Rohrleitungssystem auf 1 bis 15 Minuten, bevorzugt 1 bis 5 Minuten, begrenzt. Dadurch wird trotz Zersetzung der Schwersieder sowie der Nebenkomponenten die geforderte TEDA-Qualität im Entnahmeteil erreicht.

In einer bevorzugten Verfahrensvariante wird das Flüssigkeitsrücklaufverhältnis am oberen Ende der Trennwand auf den Zulauf- bzw. Entnahmeteil der Kolonne im Verhältnis von 1:1 bis 5, bevorzugt im Verhältnis von 1 : 1,4 bis 2 geregelt. Dies erfolgt bevorzugt in der Weise, dass die Flüssigkeit am oberen Ende der Trennwand gesammelt und über eine Regelungs- oder Stellvorrichtung im genannten Verhältnis auf den Zulauf- bzw. Entnahmeteil der Kolonne aufgegeben wird. Dadurch wird ein niedrigerer Energieverbrauch gewährleistet.

Das erfindungsgemäße Verfahren wird bevorzugt bei einem Druck im Kolonnenkopf von 0,5 bis 5 bar, bevorzugt 0,5 bis 1,5 bar, durchgeführt.

Bevorzugt ist im oberen gemeinsamen Kolonnenbereich eine Temperaturregelung vorgesehen, mit einer Messstelle unterhalb des obersten theoretischen Bodens, bevorzugt im dritten theoretischen Boden von oben gezählt, die als Stellgröße den Destillatstrom, das Rücklaufverhältnis oder bevorzugt die Rücklaufmenge, nutzt. Dadurch wird ein stabiler Betrieb der Kolonne gewährleistet mit der Folge einer weiteren Verbesserung der erzielbaren Produktreinheit.

In einer weiteren Verfahrensvariante ist zusätzlich oder alternativ eine Temperaturregelung im unteren Kolonnenbereich des Zulaufteils vorgesehen mit einer Messstelle oberhalb des untersten theoretischen Bodens, bevorzugt auf dem zweiten theoretischen Boden von unten gezählt, die als Stellgröße die Sumpfentnahmemenge nutzt. Durch diese zusätzliche Maßnahme wird eine weitere Verbesserung des stabilen Kolonnenbetriebs erreicht.

Darüber hinaus kann zusätzlich oder alternativ eine Standregelung am Kolonnensumpf im Zulauf- und/oder im Entnahmeteil vorgesehen werden, die als Stellgröße die Seitenentnahmemenge nutzt.

Die in dem erfindungsgemäßen Verfahren eingesetzte Trennwandkolonne weist eine Anzahl von theoretischen Böden von etwa 20 bis 70, bevorzugt von 30 bis 50 auf.

Bevorzugt ist die Zulaufstelle für das Roh-TEDA auf einem theoretischen Boden zwischen dem 5 bis 30., bevorzugt zwischen dem 10 bis 20. theoretischen Boden angeordnet.

Die Seitenentnahmestelle für das Rein-TEDA erfolgt zwischen dem 2 bis 20., bevorzugt zwischen dem 3 bis 20. theoretischen Boden.

Die Trennwand ist in der Kolonne bevorzugt zwischen dem Kolonnensumpf und dem 30.theoretischen Boden von unten gezählt, besonders bevorzugt zwischen dem Kolonnensumpf bis 20. theoretischen Boden angeordnet. Hierbei ist die Trennwand in einer bevorzugten Ausführungsform mittig angeordnet.

Bezüglich der trennwirksamen Einbauten gibt es grundsätzlich keine Einschränkungen; bevorzugt sind geordnete Packungen oder Böden vorgesehen.

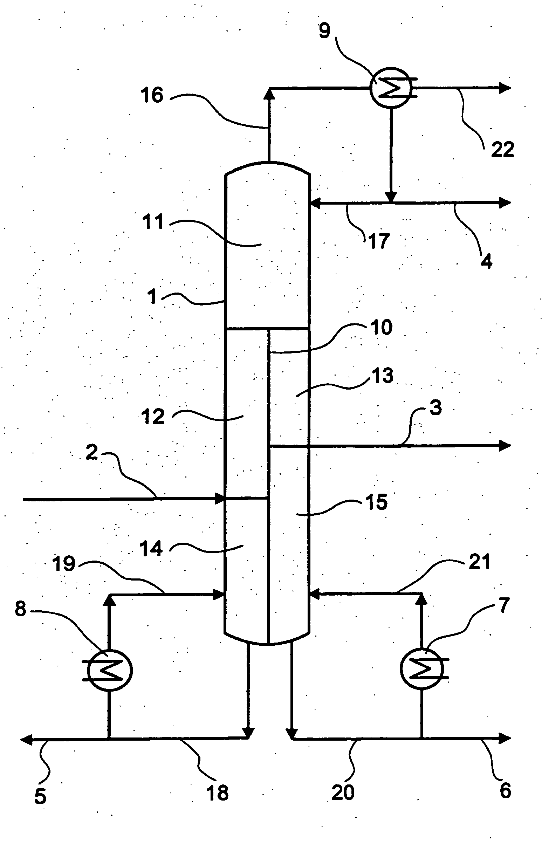

Die Erfindung wird im Folgenden anhand der Abbildung und eines Ausführungsbeispiels näher erläutert.

Die Abbildung zeigt eine Trennwandkolonne (1) mit einer Trennwand (10), welche die Trennwandkolonne in einen gemeinsamen oberen Kolonnenbereich (11), einen Zulaufteil (12), (14), mit Verstärkungsteil (12) und Abtriebsteil (14) einen Entnahmeteil (13), (15) mit einem Abtriebsteil (15) und einem Verstärkungsteil (13) unterteilt. Das Roh-TEDA tritt über die Zulaufleitung (2) zwischen den Kolonenteilen 12 und 14 in die Trennwandkolonne ein. Das Rein-TEDA wird über den Seitenabzug (3) zwischen den Kolonnenteilen 13 und 15, bevorzugt in dampfförmiger Form entnommen. Der am Kolonnenkopf anfallende Brüdenstrom wird über Leitung (16) im Kondensator (9) der gegebenenfalls durch einen Nachkühler ergänzt wird, teilweise kondensiert und in den Rücklaufstrom (17) sowie den Destillatstrom (4) aufgeteilt. Der nicht kondensierte Anteil aus dem Kondensator (9) enthält die leichtsiedenden Verunreinigungen und wird dampfförmig über Leitung (22) abgezogen. Am unteren Kolonnenende des Zulauf- und des Entnahmeteils wird die Flüssigkeit über die Leitungen (18), (20) in den Verdampfern (7), (8) teilweise verdampft und über die Rohrleitungen (19), (21) in die Kolonne zurückgeführt. Mittels Leitungen (5), (6) werden schwersiedende Verunreinigungen abgezogen. Die Verdampfer (7), (8) können als Naturumlaufverdampfer oder als Zwangsumlaufverdampfer ausgebildet sein, im letzteren Fall sind zusätzlich Umlaufpumpen für die Flüssigkeitsströme erforderlich. Besonders vorteilhaft hinsichtlich der Vermeidung von unerwünschten Zersetzungsprodukten ist es, anstelle von Zwangsumlaufverdampfern Fallfilmverdampfer oder Dünnschichtverdampfer einzusetzen, da mit dieser Bauart die kürzeren Verweilzeiten möglich sind. Zur Verringerung der Verweilzeiten der Flüssigkeit im Verdampfersystem ist es günstig, die Standhaltung nicht in der unteren Kolonnenhaube, sondern in den Zulaufleitungen der Flüssigkeit (18), (20) anzuordnen.

Das erfindungsgemäße Verfahren ermöglicht eine verfahrenstechnisch einfache, wirtschaftliche und effiziente Gewinnung reinem Triethylendiamin (TEDA) und Lösungen hiervon. Dabei werden Produkte (TEDA) von hoher Qualität bezüglich Farbe, Farbstabilität, Geruch und Reinheit hergestellt.

### Beispiel 1

Ein Roh-TEDA-Strom von 280 kg/h mit einer Temperatur von 167°C wurde flüssig auf dem 8. theoretischen Boden einer Trennwandkolonne 1 mit insgesamt 30 theoretischen Böden eingespeist. Das Roh-TEDA wies folgende Zusammensetzung auf:

| | |
|---|---|
| Wasser: | 0,1 Gew.-% |
| Leichtsieder | 1,4 Gew.-% |
| Piperazin: | 34,0 Gew.-% |
| Ethylpiperazin: | 0,9 Gew.-% |
| TEDA: | 58,2 Gew.-% |
| Aminoethylpiperazin: | 2,0 Gew.-% |
| Rückstand: | 3,4 Gew.-% |

Die Trennwand 10 erstreckte sich vom Kolonnensumpf bis zum 20. theoretischen Boden. Die Seitenentnahme 3 erfolgte auf dem 3. theoretischen Boden. Der Betrieb der Kolonne erfolgte bei einem Kopfdruck von 1,2 bar und einem Sumpfdruck von 1,3 bar. Am Kopf der Kolonne wurde bei einer Temperatur von 154°C kondensiert. Aus dem Kondensator 9 wurde ein dampfförmiger leichtsiederhaltiger Strom 22 von 4 kg/h abgezogen. Aus dem kondensierten Strom wurde ein Teilstrom 4 von 100 kg/h abgezogen. Die hochsiedenden Verunreinigungen 5, 6 wurden am Kolonnensumpf in einem Mengenstrom von 19 kg/h bei einer Temperatur von 230 bzw. 180°C entnommen. Am Seitenabzug 3 wurde das Wertprodukt TEDA bei einer Temperatur von 181°C dampfförmig in einer Menge von 157 kg/h mit einem Gehalt von 99,9 Gew.-% erhalten.
Das Aufteilungsverhältnis für die Flüssigkeit am oberen Ende der Trennwand 10 betrug 1 : 1,5 zwischen Zulauf- und Entnahmeteil.

### Beispiel 2

Wie in Beispiel 1 beschrieben, jedoch mit einem Roh-TEDA-Strom von 500 kg/h. Aus dem Kondensator 9 wurde ein dampfförmiger leichtsiederhaltiger Strom 22 von 5 kg/h abgezogen. Aus dem kondensierten Strom wurde ein Teilstrom 4 von 180 kg/h abgezogen. Die hochsiedenden Verunreinigungen 5, 6 wurden am Kolonnensumpf in einem Mengenstrom von 32 kg/h bei einer Temperatur von 230 bzw. 180°C entnommen. Am Seitenabzug 3 wurde das Wertprodukt TEDA bei einer Temperatur von 181°C dampfförmig in einer Menge von 283 kg/h mit einem Gehalt von 99,9 Gew.-% erhalten. Das Aufteilungsverhältnis für die Flüssigkeit am oberen Ende der Trennwand 10 betrug 1 : 1,5 zwischen Zulauf- und Entnahmeteil.

### Beispiel 3

Ein Roh-TEDA-Strom von 100 kg/h mit einer Temperatur von 167°C wurde flüssig auf dem 4. theoretischen Boden einer Trennwandkolonne 1 mit insgesamt 24 theoretischen Böden eingespeist. Das Roh-TEDA wies folgende Zusammensetzung auf:

| | |
|---|---|
| Wasser: | 0,1 Gew.-% |
| Leichtsieder | 1,4 Gew.-% |
| Piperazin: | 50,0 Gew.-% |
| Ethylpiperazin: | 0,9 Gew.-% |
| TEDA: | 42,2 Gew.-% |
| Aminoethylpiperazin: | 2,0 Gew.-% |
| Rückstand: | 3,4 Gew.-% |

Die Trennwand 10 erstreckte sich vom Kolonnensumpf bis zum 18. theoretischen Boden. Die Seitenentnahme 3 erfolgte auf dem 2. theoretischen Boden. Der Betrieb der Kolonne erfolgte bei einem Kopfdruck von 1,2 bar und einem Sumpfdruck von 1,3 bar. Am Kopf der Kolonne wurde bei einer Temperatur von 154°C kondensiert. Aus dem Kondensator 9 wurde ein dampfförmiger leichtsiederhaltiger Strom 22 von 4 kg/h abgezogen. Aus dem kondensierten Strom wurde ein Teilstrom 4 von 50 kg/h abgezogen. Die hochsiedenden Verunreinigungen 5, 6 wurden am Kolonnensumpf in einem Mengenstrom von 9 kg/h bei einer Temperatur von 230 bzw. 180°C entnommen. Am Seitenabzug 3 wurde das Wertprodukt TEDA bei einer Temperatur von 181 °C dampfförmig in einer Menge von 37 kg/h mit einem Gehalt von 99,9 Gew.-% erhalten.
Das Aufteilungsverhältnis für die Flüssigkeit am oberen Ende der Trennwand 10 betrug 3 : 1 zwischen Zulauf- und Entnahmeteil.

Durch das erfindungsgemäße Verfahren kann die Destillation von Roh-TEDA zu Rein-TEDA unter Einhaltung der geforderten Spezifikation mit einer Investitionskosteneinsparung von 20 % gegenüber dem herkömmlichen zweistufigen Destillationsverfahren durchgeführt werden.

## Patentansprüche

1. Verfahren zur destillativen Reinigung von Triethylendiamin (TEDA), **dadurch gekennzeichnet, dass** man die Auftrennung in einer Trennwandkolonne, deren innenliegendes Trennblech nach unten bis in den Sumpf geführt wird, sodass im unteren Kolonnenbereich getrennte Räume ausgebildet werden durchführt.

2. Verfahren nach Anspruch 1, **dadurch gekennzeichnet, dass** man das Flüssigkeitsrücklaufverhältnis am oberen Ende der Trennwand auf den Zulauf bzw. Entnahmeteil der Kolonne im Verhältnis von 1 zu 1 bis 1 zu 5 regelt.

3. Verfahren nach Ansprüchen 1 bis 2, **dadurch gekennzeichnet, dass** man die Verweilzeit im Sumpfverdampfer auf 1 bis 15 Minuten begrenzt.

4. Verfahren nach Ansprüchen 1 bis 3, **dadurch gekennzeichnet, dass** man die Trennwandkolonne bei einem Kopfdruck von etwa 0,5 bis 5 bar betreibt.

5. Verfahren nach Ansprüchen 1 bis 4, **dadurch gekennzeichnet, dass** man die Zulaufstelle für das Roh-TEDA zwischen dem fünften und dreißigsten theoretischen Boden und die Seitenentnahme für das Rein-TEDA zwischen dem zweiten und zwanzigsten theoretischen Boden positioniert.

6. Verfahren nach Ansprüchen 1 bis 5, **dadurch gekennzeichnet, dass** man die Trennwand in der Kolonne mittig zwischen dem Kolonnensumpf und dem dreißigsten Boden anordnet.

## Claims

1. A process for purifying triethylenediamine (TEDA) by distillation, wherein the fractionation is carried out in a dividing wall column
whose internal dividing wall continues down to the bottom so as to form separate chambers in the lower region of the column.

2. The process according to claim 1, wherein the ratio of runback at the upper end of the dividing wall into the inflow and offtake sections of the column is regulated in the range from 1 : 1 to 1 : 5.

3. The process according to any of claims 1 to 2, wherein the residence time in the bottom vaporizer is limited to from 1 to 15 minutes.

4. The process according to any of claims 1 to 3, wherein the dividing wall column is operated at a pressure at the top of from about 0.5 to 5 bar.

5. The process according to any of claims 1 to 4, wherein the feed point for the crude TEDA is positioned between the 5th and 30th theoretical plate and the side offtake for the pure TEDA is positioned between the 2nd and 20th theoretical plate.

6. The process according to any of claims 1 to 5, wherein the dividing wall is positioned centrally in the column between the bottom of the column and the 30th plate.

## Revendications

1. Procédé de purification par distillation de triéthylènediamine (TEDA), **caractérisé en ce qu'**on effectue la séparation dans une colonne à paroi séparatrice dont une plaque séparatrice située à l'intérieur est guidée vers le bas jusque dans le fond de façon que des espaces séparés soient formés dans la zone inférieure de la colonne.

2. Procédé suivant la revendication 1, **caractérisé en ce qu'**on règle le rapport entre le reflux de liquide à l'extrémité supérieure de la paroi séparatrice et l'amenée ou respectivement la partie de prélèvement de la colonne dans une relation de 1 à 1 jusqu'à 1 à 5.

3. Procédé suivant les revendications 1 ou 2, **caractérisé en ce qu'**on limite le temps de séjour dans l'évaporateur de fond à 1 jusqu'à 15 minutes.

4. Procédé suivant les revendications 1 à 3, **caractérisé en ce qu'**on met en service la colonne à paroi séparatrice à une pression de tête d'environ 0,5 à 5 bars.

5. Procédé suivant les revendications 1 à 4, **caractérisé en ce qu'**on positionne l'emplacement d'amenée pour le TEDA brut entre le cinquième et le trentième plateau théorique et le prélèvement latéral pour le TEDA pur entre le deuxième et le vingtième plateau théorique.

6. Procédé suivant les revendications 1 à 5, **caractérisé en ce qu'**on agence la paroi séparatrice dans la colonne au centre entre le fond de colonne et le trentième plateau.
